# EUROPEAN PATENT APPLICATION

(11) **EP 1 902 698 A1**
(43) Date of publication of application: **26.03.2008**
(21) Application number: 06019872.8
(22) Date of filing: 22.09.2006
(51) Int. Cl.: A61F 13/62

(54) **Landing zone for mechanical fasteners of disposable products, process for manufacturing thereof and process for manufacturing disposable products**

(71) Applicant: 3M Innovative Properties Company, St. Paul, Minnesota 55133-3427 (US)
(72) Inventor: Hertlein, Thomas, 41352 Korschenbroich (DE)
(74) Representative: Vossius & Partner

(57) **Abstract**

The present invention relates to a process for manufacturing a composite web for a use as landing zones for mechanical fasteners of disposable products, e.g., disposable diapers. The process comprises the steps of providing a first web material having first and second opposite surfaces, wherein the first surfaces engageable with the mechanical fastener component, providing a second web material comprising one or more indicia, wherein the second web material has a width smaller than the width of the first web material, and connecting the second web material with the second surface of the first web material to form a composite web. The invention also relates to a landing zone for mechanical fasteners and to processes for manufacturing disposable products and to disposable products as such.

## Description

### Field of the Invention

The present invention relates to a process for manufacturing a composite web for use as landing zones for mechanical fasteners of disposable products, e.g., disposable diapers and adult incontinence articles, a landing zone for mechanical fasteners of disposable products, a process for manufacturing disposable products and a disposable product with such a landing zone.

### Background Art

A disposable diaper typically comprises an absorbent core that is arranged between a backsheet and a top sheet. In an open state, the diaper has a generally hour-glass shape. The diaper comprises a rear waistband portion and a front waistband portion which are connected by an intermediate portion comprising a crotch region, whereby the waistband portions comprise side panels. The diaper furthermore comprises fastener portions or fastening tabs which are secured through their manufacturer's ends to the side panels, while user's ends can be manipulated by a user and secured to a landing zone arranged on the front waistband portion on the exposed outer surface of the backsheet when applying the diaper to a wearer. Such a diaper is schematically illustrated and described, e.g., in WO 03/028605. Similar constructions are described, e.g., in EP 0 341 993 A1, US 5 611 791, US 5 616 394, US 5 904 793, EP 1 164 007 A1, and US 5 643 397, the contents of which are incorporated herein by reference.

Typically, the fastener portion or fastening tab comprises an adhesive and/or mechanical fastener element which is adapted to be engaged with the landing zone by adhesion and/or mechanical engagement. In case of a mechanical fastening system, e.g., a hook-and-loop fastening system, the fastener portions typically comprise a hook component and the landing zone comprises a loop component. Typically, the landing zone or the fastener component of the landing zone is translucent or transparent so that an image underneath the landing zone or the fastener component of the landing zone is visible therethrough. Such an image may be provided on the backsheet of the diaper itself or in the form of a printed film (i.e. a print film) that is arranged underneath the landing zone or the fastener components of the landing zone. In known diapers, the print film and the landing zone have the same dimensions.

### Summary of the Invention

It is an object of the present invention to provide a process for manufacturing a composite web for use as landing zones for mechanical fasteners of disposable products, to provide improved landing zones for mechanical fasteners of disposable products, and to provide improved processes for manufacturing disposable products. These objects are achieved with features of the claims.

According to a first aspect, the present invention relates to a process for manufacturing a composite web for use as landing zones for mechanical fasteners of disposable products. The process comprises the steps of providing a first web material having first and second opposite surfaces, wherein the first surface is engageable with a mechanical fastener component, providing a second web material comprising one or more indicia (e.g. graphics, colorings, images or the like), wherein the second web material has a width smaller than the width of the first web material, and connecting the second web material with the second surface of the first web material to form a composite web.

Preferably, the width of the second web material is 10% to 90%, more preferably 15% to 70%, and even more preferably 20% to 60% of the width of the first web material. The width of the first web material is typically in the range of from 100mm to 300mm, preferably 175mm to 250mm, and more preferably 190mm to 220mm. For example, the width of the second web material may be in the range of from 20mm to 120mm, preferably 40mm to 100mm, and more preferably 50mm to 80mm. The width of the indicia preferably substantially corresponds to the width of the second web material. The term "substantially" in this context encompasses embodiments in which the second web material includes opposing edge regions that are free of indicia and have a width of several millimeters (e.g., 5 mm) to address different print motives on the second web material or more stable slitting process. However, it is also feasible that the width of the indicia is substantially smaller than the width of the second web material.

Preferably, the second web material comprises a printed polymeric film. According to a preferred embodiment, a plurality of second web materials is provided and connected to the first web material in several lanes, wherein the overall width of the plurality of second web materials is smaller than the width of the first web material.

The step of connecting the second web material with the second surface of the first web material preferably comprises laminating the first and second web materials together. Such lamination may be done, e.g., by extrusion lamination, adhesive lamination, thermobonding and/or calendering. The connection can be made over the entire surface of the first and second web materials or can be discontinuous, e.g., in the form of a pattern like strips, dots or the like. Another option for connecting the first and second web materials is a swirl coating process, in which threads of adhesive are applied while rotating nozzles above the web.

It is also preferred that the process comprises the further step of coating the exposed surface of the second web material with an adhesive. In addition, it is preferable to provide the exposed second surface of the first web material, i.e. the surface which is not covered by the second web material, with an adhesive. Optionally, the first surface of the first web material may be provided with an low adhesion backsize (LAB) coating that facilitates winding the composite web up to a roll.

The second web material may be provided with a non-linear edge. Moreover, it is preferred that the composite web comprises registration marks for identifying individual landing zones on the web. Such registration marks may be provided by means of the above-mentioned non-linear edges. The registration marks may also be printed on the second web material, e.g., with an ink, preferably an ultraviolet ink.

According to a preferred embodiment; the second web material is arranged approximately at the center of the first web material along the length of the webs. In other words, a center line of the second web material coincides with the center line of the first web material.

If more than one second web material is provided, it is advantageous to longitudinally cut the plurality of second web materials from a single wider web material. Such longitudinally cut second web materials may be stored on rolls and later provided in the form of several lanes to the first web material.

The process may comprise the additional step of winding the composite web into a roll from which individual landing zones can be cut transversally.

According to a further aspect, the invention relates to a landing zone for mechanical fasteners of disposable products, comprising a backing having first and second opposite surfaces, wherein the first surface is engageable with a mechanical fastener component, and an indicia carrying sheet having a width smaller than the width of the backing and being connected to the second surface of the backing.

The width of the indicia carrying sheet is preferably 10% to 90%, more preferably 15% to 70%, and even more preferably 20% to 60% of the width of the backing. The width of the backing is preferably 150mm to 300mm, more preferably 175mm to 250mm, and even more preferably 190mm to 220mm. For example, the width of the indicia carrying sheet may be 20mm to 120mm, preferably 40mm to 100mm, and more preferably 50mm to 80mm. The width of the indicia may substantially correspond to the width of the indicia carrying film. As already stated above with respect to the process aspect of the invention, the term "substantially" in this context encompasses embodiments in which an unprinted edge is provided at the longitudinal edges of the indicia carrying sheet, which may facilitate proper cutting operations and/or may compensate for sheet movement in cross direction during manufacturing of the landing zone. It is, however, also feasible to provide substantially smaller indicias on the indicia carrying film.

Preferably, the indicia carrying sheet comprises a printed polymeric film. Advantageously, a plurality of indicia carrying sheets is provided and connected to the backing in several lanes, wherein the overall width of the plurality of indicia carrying sheets is smaller than the width of the backing.

It is also preferred that the exposed surface of the indicia carrying sheet and/or the exposed second surface of the backing comprises an adhesive for attachment of the landing zone to the disposable article. The first surface of the backing may be provided with an LAB coating. Furthermore, it is preferred that the indicia carrying sheet comprises a non-linear edge. Registration marks may be printed on the indicia carrying sheet, preferably with an ink, such as an ultraviolet ink. Preferably, the indicia carrying sheet is arranged approximately at the center of the backing. If several sections of the indicia carrying sheet are present, these sections are preferably applied in a symmetrical manner.

In a still further aspect of the invention, a roll of material comprising a plurality of the landing zones as described above in endless form is provided.

A further aspect of the invention relates to a process for manufacturing disposable products, wherein the process comprises the steps of providing a continuous web of a plurality of product chassis, providing a roll of material comprising a plurality of the above described landing zones in endless form, cutting individual landing zones from the roll, and attaching a landing zone to each of the product chassis.

An alternative process for manufacturing disposable products, in which the landing zone is laminated in the disposable product manufacturing line, comprises the steps of providing a continuous web of a plurality of product chassis, providing a first web material having first and second opposite surfaces, wherein the first surface is engageable with the mechanical fastener component, providing a second web material comprising one or more indicia, wherein the second material has a width smaller than the width of the first web material, connecting the second web material in a continuous manner or in sections with the second surface of the first web material to form a composite web, cutting sections from the composite web and supplying the sections to the product chassis, and attaching a section of the composite web to each of the product chassis.

Alternatively, the process for manufacturing disposable products comprises the steps of providing a continuous web of a plurality of product chassis, providing a first web material having first and second opposite surfaces, wherein in the first surface is engageable with a mechanical fastener component, providing a second web material comprising one ore more indicia, wherein the second web material has a width smaller than the width of the first web material, supplying sections of the second web material and attaching a section to each of the product chassis, supplying sections of the first web material or supplying the first web material in a continuous manner with the second surface toward the sections of the second web material and/or the product chassis, and attaching the sections of the first web material to the sections of the second web material and/or the product chassis.

In the above-described processes, it may be preferable to wind the product chassis into a roll. The processes may comprise the additional step of cutting the product chassis transversally to form individual disposable products.

Another aspect of the invention is directed to a disposable product comprising a product chassis and a landing zone as described above. The disposable product may be manufactured according to any of the above-mentioned manufacturing processes.

The landing zone of the present invention is particularly advantageous over the prior art in that it provides an easy and reliable means for properly positioning the fastener portion of the diaper on the landing zone. In addition, the landing zones according to the invention are easier and cheaper to manufacture, while still being very attractive. Moreover, with the landing zone according to the invention it is possible to provide indicia carrying areas only under certain parts of the landing zone.

The print film that is typically used as an indicia carrying sheet adds substantially to the overall costs in a manufacture of landing zones. Typically, the print film is not needed as a carrier for the mechanical fastener component of the landing zone, but only as a carrier for a print design. Accordingly, the inventors discovered that providing the indicia carrying sheet only at certain areas of the landing zone, e.g., the center portion, is sufficient for most applications. The reduction of film material directly leads to a significant cost saving. Another benefit is the increase of flexibility and/or breathability in the area of the landing zone edges.

The processing of the adhesive carrying sheets/second web material having a width smaller than the width of the backing/first web material at exactly the position that is needed provides several advantages in regard to cross-machine direction (CD) registration, particularly when a plurality of second web materials are provided. In addition, shrinkage of the second web material during lamination will not cause registration problems as with known techniques in which the first and second web materials have substantially the same width. The shrinkage is essentially caused by web tension in combination with process temperature so that according to known processes films with good temperature resistance and high strength are required. This requirement is typically accomplished by high caliper films. Another disadvantage of the current technology is that each size or width of a finished landing zone needs an individual print design. According to the invention, it is possible to use an indicia carrying sheet of unitary width for all feasible widths of backing sheets. According to the invention it is also possible that the first web material forms the backsheet of the disposable product.

### Brief Description of the Drawings

In the following, preferred embodiments of the present invention will be described with reference to the drawings, in which
- Fig. 1: is a schematic representation of a disposable diaper in a closed form;
- Fig. 2: is a schematic cross-section of an indicia carrying sheet attached to a backing that has a first surface being engageable with a mechanical fastener component;
- Fig. 3: is a schematic cross-section through a landing zone according to an embodiment of the invention;
- Fig. 4: is a schematic cross-section through a landing zone according to another embodiment of the invention;
- Fig. 5a: is a schematic cross-section through a landing zone according to a further embodiment of the invention during manufacturing thereof;
- Fig. 5b: shows the final landing zone according to the embodiment of Fig 5a in the final state;
- Fig. 6: is a schematic cross-section through a landing zone according to another embodiment of the invention;
- Fig. 7: shows a schematic top view on a composite web material according to the invention;
- Fig. 8: is a schematic illustration of a process for manufacturing a disposable diaper according to the present invention;
- Fig. 9: is a schematic illustration of another process for manufacturing a disposable diaper according to the invention; and
- Fig. 10: is a schematic illustration showing how a plurality of indicia carrying sheets may be arranged next to each other.

### Detailed Description of Preferred Embodiments

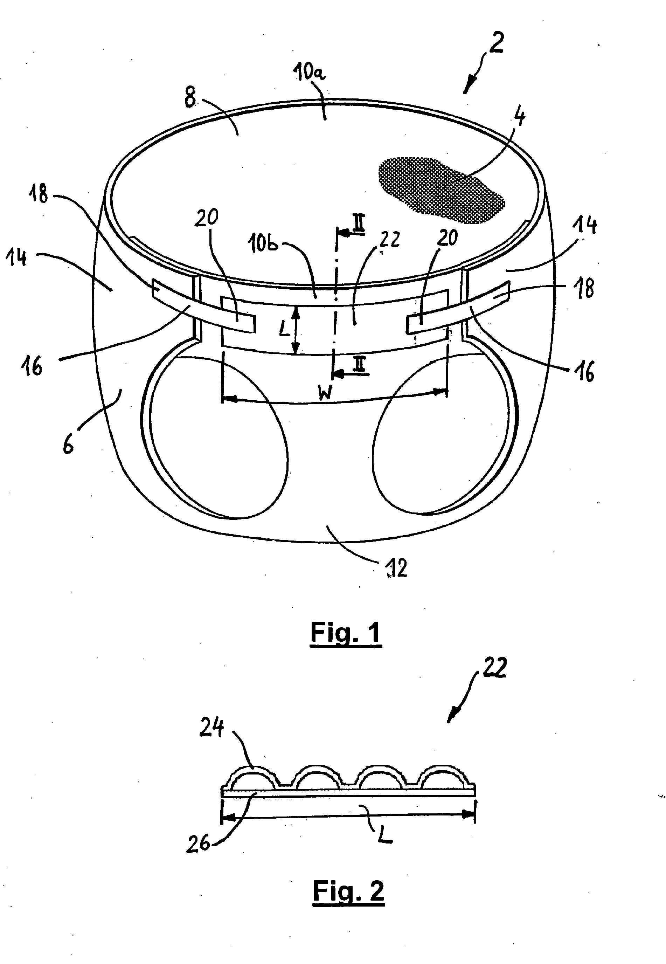

Fig. 1 is a schematic representation of a disposable product in the form of a disposable diaper 2. The diaper 2 comprises an absorbent core 4 that is arranged between a backsheet 6 and a top sheet 8. The diaper 2 comprises a rear waistband portion 10a and a front waistband portion 10b which are connected by an intermediate portion comprising a crotch region 12, whereby the waistband portions 10a, 10b comprise side panels 14. The diaper 2 furthermore comprises fastener portions or fastening tabs 16 which are secured through their manufacturer's ends 18 to the side panels 14, while their user's ends 20 can be manipulated by a user and secured to a landing zone 22 when applying the diaper 2 to a wearer.

Fig. 2 is a schematic illustration of the landing zone 22 in a cross-sectional view along line II-II of Fig. 1. As can be seen from the cross-sectional view according to Fig. 2, the landing zone 22 comprises a backing 24 having first and second opposite surfaces, wherein the first surface is engageable with a mechanical fastener component, e.g., a hook element of a hook-and-loop fastening system. The backing 24 is formed from a first web material. First web materials that are useable in the landing zone of the present invention are known to those skilled in the art and disclosed, e.g. in EP 0 341 993 A1, US 5 611 791, US 5 616 394, US 5 643 397, US 5 904 793, EP 1 164 007 A1, WO 98/26684, WO 98/48751, EP 1 172 472 A1, and US 5 133 707, the contents of which are incorporated herein by reference. In accordance with the invention, it would also be possible to use the backsheet 6 of the diaper 2 as a first web material as disclosed, e.g., in GB 2 327 857 A, the contents of which are incorporated herein by reference.

The landing zone 22 furthermore comprises an indicia carrying sheet 26 formed from a second web material. The indicia carrying sheet 26 comprises one or more indicia (not shown), e.g., graphics, colorings, images, letters, numbers, etc., and is also referred to as indicia carrying sheet. The indicia carrying sheet 26 is preferably formed as a printed polymeric film and is connected to the second surface of the backing 24. The connection between the backing 24 and the indicia carrying sheet 26 is typically provided by lamination, e.g., extrusion lamination, adhesive lamination, thermobonding, calendering and/or ultrasonic bonding. The bonding between the backing 24 and the indicia carrying sheet 26 can be continuous over the entire surface or discontinuous, e.g., in a pattern, like strips, dots or the like. Another method for connecting the backing 24 and the indicia carrying sheet 26 is swirl coating.

The length L in the machine direction (MD) of the landing zone 22 is typically in the range of from 10mm to 100mm, preferably 25mm to 60mm. The width W in the cross-machine direction (CD) of the landing zone 22 is typically 150mm to 300mm, preferably 175mm to 250mm and more preferably 190mm to 220mm for a disposable diaper.

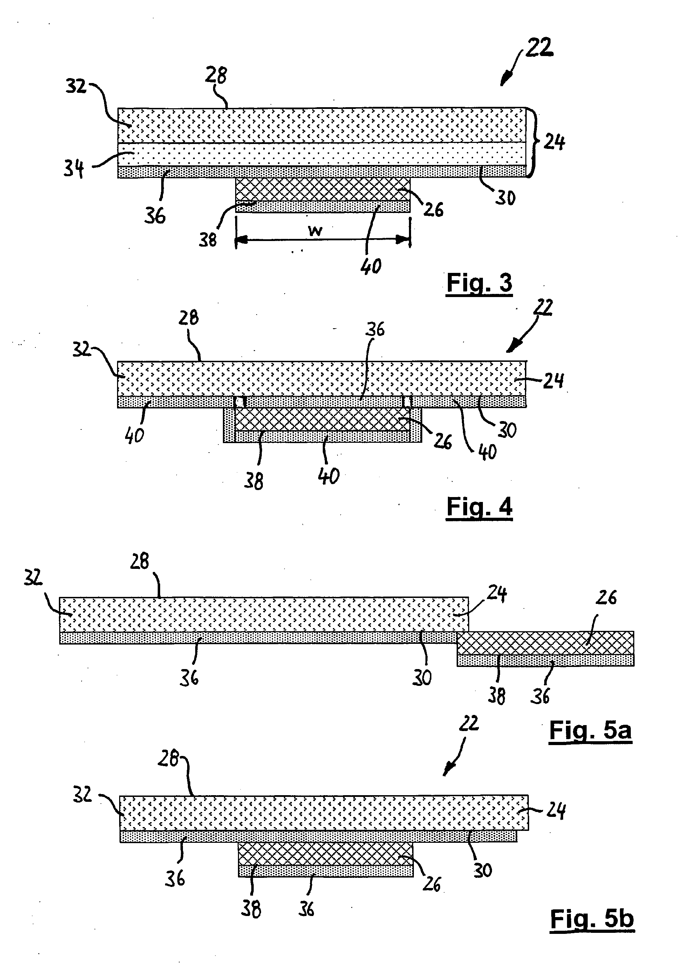

Figs. 3 to 5 show different embodiments of the landing zone 22 of the present invention in cross-section along the cross-machine direction. In the embodiment according to Fig. 3, the landing zone 22 comprises a backing 24 having first and second opposite surfaces 28, 30. The first surface 28 of the backing 24 is engageable with a mechanical fastener component, e.g., a hook patch provided on the fastening tab 16 of the diaper 2. In the embodiment shown in Fig. 3, the first surface 28 of the backing 24 is provided with a loop material 32, wherein the second surface 30 of the backing 24 is provided with an extrudate layer 34 in which the loops of the loop material 32 are anchored. Such a loop material is described in more detail, e.g., in EP 0 341 993 A1, US 5 611 791, US 5 616 394, US 5 643 397, US 5 904 793 and EP 1 164 007 A1, the contents of which are incorporated herein by reference. The second surface 30 of the backing 24 is preferably provided with an adhesive layer 36 by means of which the indicia carrying sheet 26 is attached to the backing 24. Alternatively, the indicia carrying sheet 26 may be attached to the backing 24 by means of strip extrusion bonding, i.e. that the indicia carrying sheet 26 is bonded to the backing 24 by means of the extrudate layer 34.

As shown in Fig. 3, the indicia carrying sheet 26 is arranged approximately at the center of the backing 24 in cross-machine direction and has a width w of about 40% of the width W of the backing 24. More generally, the width w of the indicia carrying sheet 26 is about 10% to 90%, preferably 15% to 70%, and more preferably 20% to 60% of the width W of the backing 24.

An exposed surface 38 of the indicia carrying sheet 26 is preferably provided with an adhesive layer 40 so that the landing zone can be attached to the backsheet 6 of the diaper 2 by means of the exposed portions of the adhesive layer 36 of the backing 24 and the adhesive layer 40 of the indicia carrying sheet 26. As mentioned above, the adhesive layers 40 or 36 may be continuous or discontinuous.

In case of a strip extrusion bonded indicia carrying sheet 26, it is preferred to provide the whole exposed surface of the backing 24 and the indicia carrying sheet 26 with an adhesive layer (similar to the embodiment of Fig. 4).

The backing 24 is preferably translucent or transparent so that any indicia provided on the indicia carrying sheet 26 is visible through the backing 24. Typically, the width of the indicia provided on the indicia carrying sheet 26 substantially corresponds to the width w of the indicia carrying sheet 26. The term "substantially" is considered in this context to encompass embodiments in which an unprinted edge region of several millimeters. Alternatively, the width of indicia provided on the indicia carrying sheet may be smaller or even substantially smaller than the width of the indicia carrying sheet 26.
In Fig. 4 another embodiment of a landing zone 22 according to the present invention is illustrated. In this embodiment, the backing 24 is formed from a single-layer material, e.g., a single-layer nonwoven material 32, instead of the multilayer construction of the embodiment according to Fig. 3. Moreover, in the embodiment according to Fig. 4, the second surface 30 of the backing 24 is provided with an adhesive layer 36 only in the area of the indicia carrying sheet 26. Accordingly, in this embodiment the indicia carrying sheet 26 is laminated to the backing 24 by means of the adhesive layer 36, thereupon the adhesive layer 40 is applied in a subsequent step to cover the exposed surface 38 of the indicia carrying sheet 26 and the exposed surfaces of the backing 24.

In Figs. 5a and 5b, the landing zone 22 is generally similar to the landing zone of the embodiment of Fig. 4, wherein the process of manufacturing the landing zone is slightly different as evident from Fig. 5a. In manufacturing the landing zone 22, the backing 24 and the indicia carrying sheet 26 are arranged with a slight overlap of, for example, several millimeters so that both the backing 24 and the indicia carrying sheet 26 can be provided with an adhesive layer 36 in a single coating step. After the coating step, the backing with its adhesive layer 36 on the exposed surface 38 is arranged in the center area of the backing 24 and is laminated to the adhesive layer 36 provided on the second surface of the backing 24.

The landing zone 22 according to the present invention is particularly advantageous in that it reduces the amount of indicia carrying sheet 26 on the backing 24 so that besides the cost saving aspect of this construction the breathability and flexibility in the landing zone 22 can be considerably improved vis-à-vis constructions where the indicia carrying sheet extends over the whole width W of the backing 24.

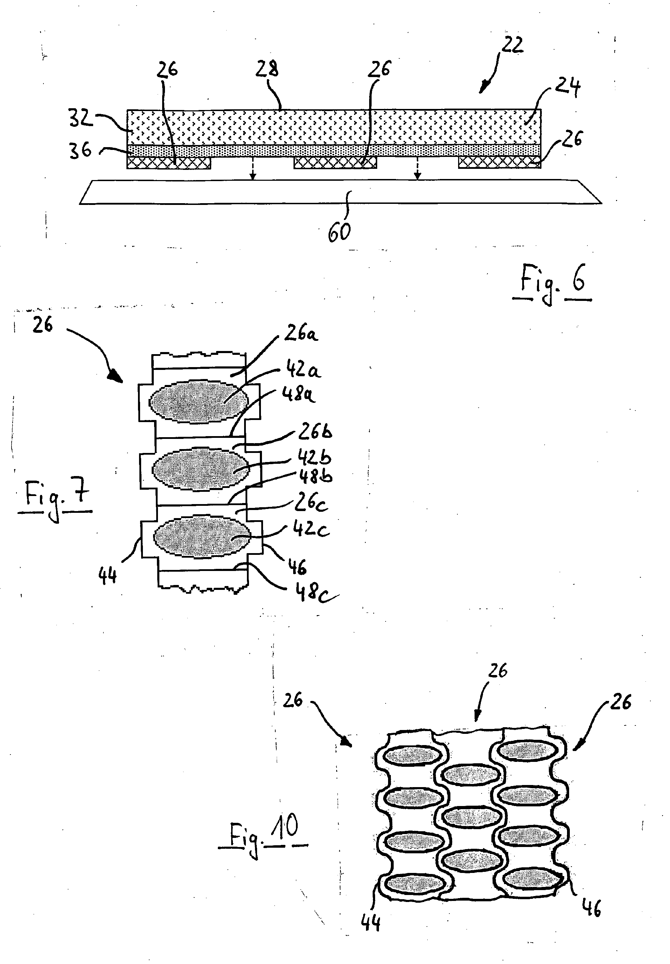

Another embodiment of the landing zone 22 of the present invention is illustrated in Fig. 6. According to this embodiment, the backing 24 is provided with an adhesive layer 36 by means of which a plurality of indicia carrying sheets 26 are attached to the backing 24. The indicia carrying sheets 26 are not provided with adhesive, but the attachment to the diaper chassis 60 is made by exposed adhesive sections being present between adjacent indicia carrying sheets 26. This is schematically illustrated in Fig. 6 by the dashed arrows.

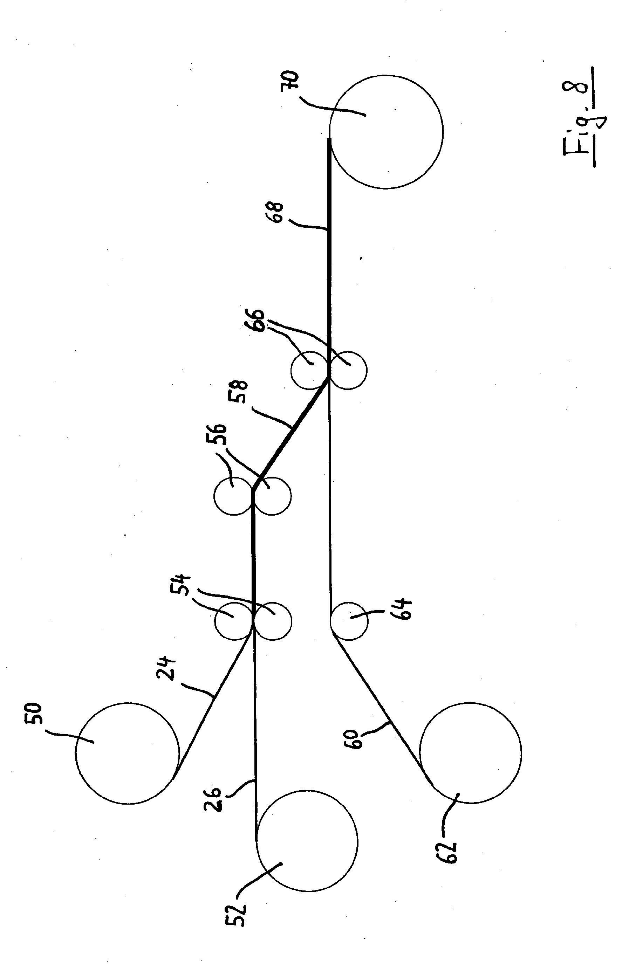

Fig. 7 shows a second web material comprising a plurality of indicia carry sheets 26a, 26b, 26c with respective indicia 42a, 42b, 42c thereon. The second web material can be used in the manufacturing of a composite web material according to the present invention from which individual landing zones 22 can be cut transversally, as will be described in the following with particular reference to Figs. 7 and 8.

The second web material shown in Fig. 7 comprises non-linear edges 44, 46 in the longitudinal or machine direction. These non-linear edges 44, 46 may be used as registration marks for identifying individual indicia carrying sheets 26 and thus landing zones 22 that are provided on the composite web. Additionally, this arrangement reduces the amount of material necessary for the production of the landing zones. By means of the registration marks a cutting operation can be initiated during a manufacturing process of the composite web and/or the diaper manufacturing process so that individual indicia carrying sheets 26a, 26b, 26c and/or landing zones 22 can be cut at an appropriate position along cut lines 48a, 48b, 48c.

Alternatively or additionally, registration marks can be printed on the second sheet material by means of an ink, preferably an ultraviolet ink that is not visible for the naked eye. In such an embodiment, the edges 44, 46 of the second web material can also be straight in a longitudinal direction.

In the following, a process for manufacturing a composite web for use as landing zones 22 for mechanical fasteners of disposable diapers and processes for manufacturing of disposable diapers will be described with reference to Figs. 7 and 8. In Fig. 8, a composite web material is manufactured from a first web material that is supplied from a first supply roll 50. The first web material forms the backing 24 of the landing zone 22. As such, the first web material has first and second opposite surfaces, as described previously, wherein the first surface 28 is engageable with the mechanical fastener component. Also provided is a second web material from a second supply roll 52. The second web material provides the indicia carrying sheets 26 of the landing zone 22. The width w of the second web material is smaller than the width W of the first web material . The first and second web materials are lead through a first pair of guide rollers 54 towards a first lamination station 56 in which the first and second web materials are connected to each other. This connection can be continuous or discontinuous in any desired pattern. In the first lamination station 56, the composite web material 58 according to the present invention is assembled.

The composite web material 58 can be wound into a roll and stored for later use or delivery to a diaper manufacturer. Alternatively, as shown in Fig. 8, the composite web 58 may be further processed in a diaper manufacturing line. To this end, an endless web 60 of a plurality of diaper chassis is provided from a further supply roll 62 and guided over a guide roller 64 towards a second lamination station 66 where individual landing zones 22 that are cut from the composite web 58 are laminated to the web 60 of diaper chassis. This web 68 of diaper chassis with attached landing zones 22 may be further processed to complete the diaper 2 or may the wound onto a storage roll 70 for later processing.

As mentioned above, it would also be possible to supply a roll of composite web 58 to the diaper manufacturing line instead of laminating the composite web 58 at the diaper manufacturing line. In such an embodiment, the composite web 58 would also be cut into sections and laminated to the web 60 of diaper chassis at a lamination station 66 in order to provide a diaper chassis with attached landing zone 22 that can be either stored on a roll 70 or further processed to a complete diaper.

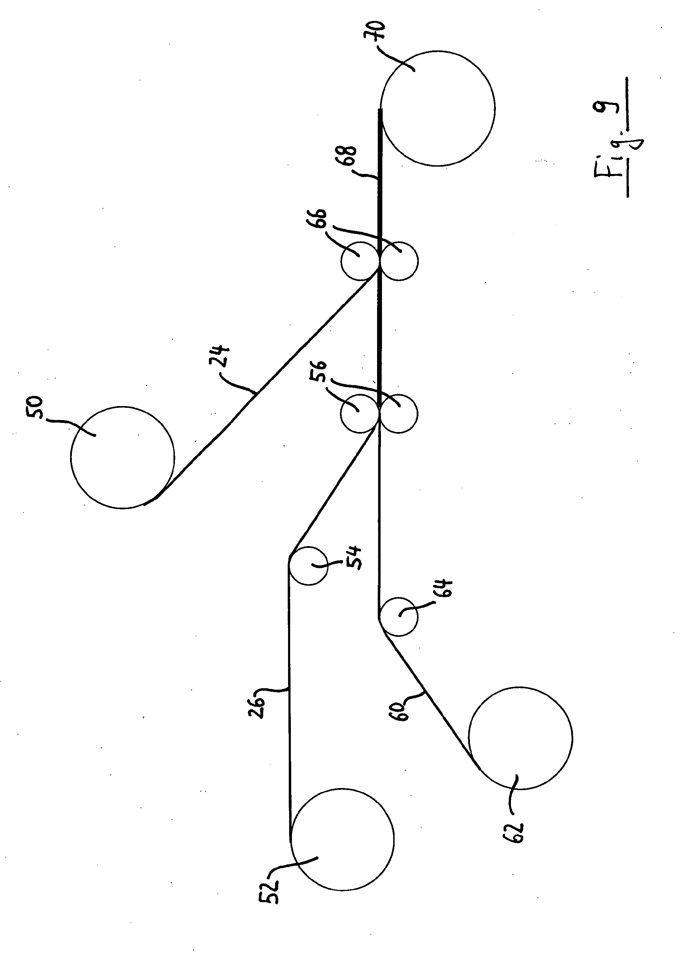

In the embodiment according to Fig. 9, a web 60 of a plurality of diaper chassis is provided from the supply roll 62 and guided over a guide roller 64 to a first lamination station 56. At the first lamination station 56, the second web material that is supplied from supply roll 52 and guided over roller 54 is cut into individual sections, i.e. indicia carrying sheets 26, and attached to the web 60 of diaper chassis at appropriate positions, which are preferably detected by the registration marks. The web is then either stored on a storage roll (not shown) or fed to a second lamination station 66 to which also the first web material is supplied from supply roll 50. At the second lamination station 66 sections of the first web material, i.e. the backing 24 of the landing zone 22, are cut and attached to the sections of the second web material and/or the product chassis. After the lamination, the web 68 of diaper chassis with landing zones 22 may be further processed to a complete diaper or may be stored on a storage roll 70.

In the process for manufacturing the composite web 58 as described above with reference to Fig. 8, it is advantageous to supply a plurality of second web materials and connect them to the first web material in several lanes. The overall width of the plurality of second web materials in such an embodiment is smaller than the width of the first web material. In accordance with such an embodiment, it is possible to provide several strips of indicia carrying sheet 26 on a single backing 24, or to provide a wide composite web from which a plurality of composite webs with individual landing zones can be cut longitudinally. The plurality of second web materials is preferably provided in the form of a single web material which is cut longitudinally.

In Fig. 10, an embodiment of a web from which a plurality of second web materials can be cut is illustrated. In this embodiment, the individual second web materials are cut longitudinally and then separated from each other in order to appropriately position the individual second web materials on the first web material. It may also be advantageous to provide an unprinted edge region of several millimeters that is cut away in order to be able to compensate for web movement.

According to the present invention it would also be possible to provide the indicia carrying sheet 26 underneath the backsheet 6 of the disposable diaper, if the backsheet 6 is sufficiently translucent or transparent that the indicia of the indicia carrying sheet 26 is visible through the backsheet 6. In such an embodiment the backsheet 6 forms the backing 24 of the landing zone 22.

As mentioned above, the landing zone according to the present invention provides several advantages over the prior art, particularly an improved breathability through the landing zone 22, improved flexibility of the disposable product in the area of the landing zone, better guidance for positioning the fastening tab 16 on the landing zone 22 and a substantial cost saving due to the reduced amount of indicia carrying sheet required for the landing zone. Moreover, the landing zone of the present invention is advantageously adapted for fastening tabs 16 and/or printed backsheets 6 and landing zones 22 which form together a complimentary design on the front waistband portion 10b of the diaper. Such complimentary print designs are described, e.g., in WO 01/21126, the contents of which are incorporated herein by reference. This arrangement means that the printed backsheet 6 forms the large size indicia (generally with low variation) and the landing zone 22 forms the smaller size indicia, e. g. to provide for more variations.

## Claims

1. A process for manufacturing a composite web for use as landing zones for mechanical fasteners of disposable products, the process comprising the steps of:
(a) providing a first web material having first and second opposite surfaces, the first surface being engageable with a mechanical fastener component;
(b) providing a second web material comprising one or more indicia, the second web material having a width smaller than the width of the first web material; and
(c) connecting the second web material with the second surface of the first web material to form a composite web.

2. The process of claim 1, wherein the width of the second web material is 10 % to 90 %, preferably 15 % to 70 %, more preferably 20 % to 60 % of the width of the first web material.

3. The process of claim 1 or 2, wherein the width of the indicia substantially corresponds to the width of the second web material.

4. The process of any of claims 1 to 3, wherein the second web material comprises a printed polymeric film.

5. The process of any of claims 1 to 4, wherein a plurality of second web materials is provided and connected to the first web material in several lanes, wherein the overall width of the plurality of second web materials is smaller than the width of the first web material.

6. The process of any of claims 1 to 5, further comprising the step of coating the exposed surface of the second web material with an adhesive.

7. The process of any of claims 1 to 6, wherein the second web material is provided with a non-linear edge.

8. The process of any of claims 1 to 7, wherein registration marks for identifying individual landing zones are provided on the composite web.

9. The process of claim 8, wherein the registration marks are provided by means of said non-linear edges.

10. The process of claim 8, wherein the registration marks are printed on the second web material, preferably with ultraviolet ink.

11. The process of any of claims 1 to 10, wherein the second web material is arranged approximately at the center of the first web material along the length of the webs.

12. The process of any of claims 5 to 11, wherein the plurality of second web materials is provided from a single web material which is cut longitudinally.

13. A landing zone for mechanical fasteners of disposable products, comprising a backing having first and second opposite surfaces, the first surface being engageable with a mechanical fastener component, and an indicia carrying sheet having a width smaller than the width of the backing and being connected to the second surface of the backing.

14. The landing zone of claim 13, wherein the width of the indicia carrying sheet is 10 % to 90 %, preferably 15 % to 70 %, more preferably 20 % to 60 % of the width of the backing.

15. The landing zone of claim 13 or 14, wherein the width of the indicia substantially corresponds to the width of the indicia carrying sheet.

16. The landing zone of any of claims 13 to 15, wherein the indicia carrying sheet comprises a printed polymeric film.

17. The landing zone of any of claims 13 to 16, wherein a plurality of indicia carrying sheets is provided and connected to the backing in several lanes, wherein the overall width of the plurality of indicia carrying sheets is smaller than the width of the backing.

18. The landing zone of any of claims 13 to 17, wherein the exposed surface of the indicia carrying sheet comprises an adhesive.

19. The landing zone of any of claims 13 to 18, wherein the indicia carrying sheet comprises a non-linear edge.

20. The landing zone of any of claims 13 to 19, further comprising a registration mark.

21. The landing zone of claim 20, wherein the registration mark is provided by means of said non-linear edge.

22. The landing zone of claim 20, wherein the registration mark is printed on the indicia carrying sheet, preferably with an ultraviolet ink.

23. The landing zone of any of claims 13 to 22, wherein the indicia carrying sheet is arranged approximately at the center of the backing.

24. A roll of material comprising a plurality of landing zones of any of claims 13 to 23 in endless form.

25. A process for manufacturing disposable products, the process comprising the steps of:
(a) providing a continuous web of a plurality of product chassis;
(b) providing a roll according to claim 24;
(c) cutting individual landing zones form said roll; and
(d) attaching a landing zone to each of said product chassis.

26. A process for manufacturing disposable products, the process comprising the steps of:
(a) providing a continuous web of a plurality of product chassis;
(b) providing a first web material having first and second opposite surfaces, the first surface being engageable with a mechanical fastener component;
(c) providing a second web material comprising one or more indicia, the second web material having a width smaller than the width of the first web material;
(d) connecting the second web material with the second surface of the first web material to form a composite web;
(e) cutting sections from the composite web and supplying said sections to said product chassis; and
(e) attaching a section of said composite web to each of the product chassis.

27. A process for manufacturing disposable products, the process comprising the steps of:
(a) providing a continuous web of a plurality of product chassis;
(b) providing a first web material having first and second opposite surfaces, the first surface being engageable with a mechanical fastener component;
(c) providing a second web material comprising one or more indicia, the second web material having a width smaller than the width of the first web material;
(d) supplying sections of the second web material and attaching a section to each of the product chassis;
(e) supplying sections of the first web material with the second surface toward the sections of second web material and/or the product chassis, and attaching the sections of the first web material to the sections of the second web material and/or the product chassis.

28. The process of any of claims 25 to 27, wherein the product chassis are wound into a roll.

29. The process of any of claims 25 to 28, wherein the product chassis are cut transversally to form individual disposable products.

30. A disposable product comprising a product chassis and a landing zone of any of claims 13 to 23.

31. A disposable product manufactured according to the process of any of claims 25 to 29.
